# EUROPÄISCHE PATENTANMELDUNG

(11) **EP 4 703 048 A1**
(43) Veröffentlichungstag der Anmeldung: **04.03.2026**
(21) Anmeldenummer: 24198143.0
(22) Anmeldetag: 03.09.2024
(51) Int. Cl.: B05C 1/06, A61L 27/00, B05C 3/10, B05D 1/18

(54) **VORRICHTUNG ZUR BESCHICHTUNG VON IMPLANTATEN UND ZUGEHÖRIGES VERFAHREN**

(71) Anmelder: Heraeus Medical GmbH, 61273 Wehrheim (DE)
(72) Erfinder: vogt, Sebastian, 61273 Wehrheim (DE); kluge, Thomas, 61273 Wehrheim (DE)
(74) Vertreter: Heraeus IP

(57) **Zusammenfassung**

Die Erfindung betrifft eine Vorrichtung zur Beschichtung von länglichen Implantaten, ein System mit einer solchen Vorrichtung, eine Verwendung einer Vorrichtung sowie ein Verfahren zur Beschichtung eines länglichen Implantats. Eine Vorrichtung (10) zur Beschichtung eines länglichen Implantats (5) umfasst einen Vorratsbehälter (12) zur Aufnahme eines Beschichtungsmittels (8) sowie ein mit dem Vorratsbehälter (12) verbundenes oder verbindbares Unterteil (15) mit einer Öffnung (16). Ein die Öffnung (16) ausbildendes Material (17) ist gummielastisch und dient als Abstreifer (18) zum Abstreifen von überschüssigem Beschichtungsmittel (8).

## Beschreibung

Die Erfindung betrifft eine Vorrichtung zur Beschichtung von länglichen Implantaten, ein System mit einer solchen Vorrichtung, eine Verwendung einer Vorrichtung sowie ein Verfahren zur Beschichtung eines länglichen Implantats.

Zur Behandlung von Knochenfrakturen, beispielsweise in der Unfallchirurgie, werden Marknägel (intramedulläre Nägel) und Osteosyntheseplatten verwendet. Dies erfolgt insbesondere bei Frakturen von Röhrenknochen wie den Oberschenkelknochen (Femur), Schienbeinknochen (Tibia) oder den Oberarmknochen (Humerus). Nach derartiger Versorgung kann es zu Infektionen des umliegenden Knochen- und/oder Weichgewebes kommen, beispielsweise mit Bakterien wie Staphyloccus aureus oder Staphylococus epdidermidis. Dies macht eine chirurgische Sanierung notwendig, bei der infiziertes Gewebe abgetragen wird. Zudem werden eine systemische Antibiose und/oder lokale Antiinfektiva eingesetzt, um die Infektion gezielt zu bekämpfen.

Es ist wünschenswert, wenn nach der chirurgischen Sanierung eine mechanische Stabilisierung des frakturierten Knochengewebes weiterhin möglich wäre, beispielsweise durch längliche Implantate wie Marknägel und/oder Osteosyntheseplatten, und diese gleichzeitig lokal Antiinfektiva abgeben könnten, um im debridierten Gewebe verbliebene mikrobielle Keime lokal zu unterdrücken.

Es ist bekannt, Marknägel mit einer Antibiotika enthaltenden Beschichtung aus Polymethylmethacrylat-Knochenzement zu verwenden (N. Walter et al.: "Individual and commercially available antimicrobial coatings for intramedullary nails for the treatment of infected long bone non-unions - a systematic review"; Injury 2022; DOI: 10.1016/j.injury.2022.05.008). Antibiotika aus dem Knochenzement werden durch wässrige Körperflüssigkeiten, wie Wundsekret und Blut, aus der Beschichtung herausgelöst und führen zu lokal hohen Antibiotika-Konzentration. In Kombination mit der systemischen Antibiose können die nach dem Debridement möglicherweise noch verbliebenen Keime reduzieren.

Wünschenswert ist es ferner, eine derartige Beschichtung intraoperativ herzustellen. Dies ermöglicht, exakt auf die vorliegenden Keime abgestimmte Antiinfektiva in das Beschichtungsmittel einzubringen. Bisher erfolgt die intraoperative Beschichtung so, dass Knochenzement mit den benötigten Antiinfektiva hergestellt wird und eine Schicht des Knochenzements manuell um den zu beschichtenden Marknagel geformt wird. Dabei ist es sehr schwierig, eine gleichmäßige Schichtdicke über die gesamte Länge des Marknagels zu erzielen. Es kommt häufig vor, dass der beschichtete Nagel mindestens teilweise einen zu großen Querschnitt aufweist und nicht mehr in den debridierten Knochen implantiert werden kann. Aufgrund der starken Haftung von Polymethylmethacrylat-Knochenzement auf Metalloberflächen kann der aufgetragenen Knochenzement nach schon kurzer Zeit nicht mehr von der Oberfläche des Marknagels entfernt werden. Auch bereichsweise zu geringe Schichtdicken kommen vor, so dass zumindest lokal zu geringe Mengen der Antiinfektiva zur Verfügung stehen.

In Druckschrift US2007/0134287A1 wird eine antibiotische Lösung zur Beschichtung beschrieben, die aus einem leicht verdampfenden Lösungsmittel und einem darin gelösten Antibiotikum besteht. Druckschrift US11517650B2 beschreibt eine ähnliche Lösung zur Beschichtung, bei der Antibiotika in einem schnell oder mittelschnell verdampfenden Lösungsmittel gelöst werden und zur Beschleunigung des Lösungsprozesses Energie durch eine Einstrahlung von Ultraschall zugeführt wird. In Druckschrift EP1243259B1 wird eine Beschichtung offenbart, die aus Polymethylmethacrylat und einem hydrophilen Polymer besteht, in der ein Antibiotikum suspendiert ist. Eine ähnliche Lösung ist in EP1112095B1 beschrieben. Hierbei wird eine Suspension wird auf Implantatoberflächen aufgetragen, wobei das Lösungsmittel verdunstet und ein D,L-Polylactidfilm zurückbleibt, in dem Antibiotikumpartikel fixiert sind.

Die oben genannten Merkmale können beliebig mit den unterschiedlichen Aspekten der Erfindung kombiniert werden.

Es ist die Aufgabe der Erfindung, längliche Implantate auf einfache Weise mit einer definierten Beschichtung zu versehen.

Die Aufgabe wird gelöst durch die Vorrichtung gemäß Anspruch 1 sowie durch das System, die Verwendung und das Verfahren gemäß den nebengeordneten Ansprüchen. Vorteilhafte Ausgestaltungen sind in den Unteransprüchen angegeben.

Zur Lösung der Aufgabe dient eine Vorrichtung zur Beschichtung eines länglichen Implantats. Die Vorrichtung umfasst einen Vorratsbehälter zur Aufnahme eines Beschichtungsmittels sowie ein Unterteil. Insbesondere ist das Unterteil mit dem Vorratsbehälter verbunden oder verbindbar. Das Unterteil weist eine Öffnung auf. Ein die Öffnung ausbildendes Material ist gummielastisch und dient als Abstreifer zum Abstreifen von überschüssigem Beschichtungsmittel.

Während der bestimmungsgemäßen Verwendung befindet sich der Vorratsbehälter oberhalb des Unterteils. Ein Beschichtungsmittel befindet sich im Vorratsbehälter. Ein längliches Implantat wird nach unten durch den Vorratsbehälter und die Öffnung hindurch bewegt, typischerweise entlang der zentralen Achse der Vorrichtung. Im Vorratsbehälter und/oder in darunter liegenden Bereichen der Vorrichtung kann das Implantat mit dem Beschichtungsmittel benetzt werden. Der Abstreifer entfernt überschüssiges Beschichtungsmittel von der Oberfläche des Implantats. Auf diese Weise wird eine gleichmäßige Beschichtung ermöglicht. Der die Öffnung ausbildende Bereich passt sich an die Form des Implantats an und ermöglicht so ein gleichmäßiges Abstreifen. Die Beschichtung kann anschließend innerhalb von Minuten aushärten.

Die Vorrichtung ist einfach und kostengünstig herstellbar, einfach in der Verwendung und erlaubt die reproduzierbare Herstellung definierter Beschichtungen. Aufgrund der einfachen und raschen Anwendung kann dies auch intraoperativ erfolgen, so dass eine Beschichtung mit einem individuell angepassten Wirkstoff verwendet werden kann.

Das Beschichtungsmittel kann beispielsweise Knochenzement oder Polymer umfassen. Alternativ oder ergänzend kann das Beschichtungsmittel einen oder mehrere Wirkstoffe wie z. B. Antiinfektiva umfassen. Wirkstoffe können in einem oder mehreren Polymeren gelöst oder suspendiert sein. Das Implantat kann beispielsweise ein Marknagel oder eine Osteosyntheseplatte sein.

Der Vorratsbehälter ist insbesondere nach oben bzw. an der der Öffnung abgewandten Seite offen. So kann das Implantat und/oder das Beschichtungsmittel von oben in den Vorratsbehälter eingeführt bzw. eingegeben werden.

Der Vorratsbehälter kann eine rotationssymmetrische Grundform aufweisen. Rotationssymmetrische Grundform meint, dass die zugrundeliegende Form rotationssymmetrisch ist. Es können an oder in dieser Grundform Anbauten, zusätzliche Teile, Aussparungen, Löcher, etc. vorhanden sein, sodass die tatsächliche Form von der rotationssymmetrischen Form abweichen kann. Es können auch anderweitige Abweichungen von der rotationssymmetrischen Form vorliegen, wie z. B. Verdickungen, Abflachungen, etc. der Vorratsbehälter umschließt einen Hohlraum zur Aufnahme des Beschichtungsmittels.

Das Unterteil ist der Bereich der Vorrichtung, der die Öffnung ausbildet. Die Öffnung dient dem Heraustreten des Implantats aus der Vorrichtung nach dem Beschichten. Die Wandung der Öffnung kann als Abstreifer ausgebildet sein. Der Abstreifer umfasst typischerweise eine Kante, die dem Abstreifen dient, und kann auch als Abstreiflippe bezeichnet werden. Der Abstreifer ist insbesondere so beschaffen, dass eine mehr oder weniger viskose Flüssigkeit nicht zwischen Abstreifer und Implantat hindurchfließen kann.

Das Unterteil ist mechanisch mit dem Vorratsbehälter verbunden oder verbindbar. Es kann eine manuelle und/oder reversible Verbindung vorgesehen sein. Die Verbindung ist insbesondere flüssigkeitsdicht. Das Unterteil kann indirekt mit dem Vorratsbehälter verbunden oder verbindbar sein.

Das die Öffnung ausbildende Material ist aus einem gummielastischen Material hergestellt. Insbesondere ist das Unterteil zumindest teilweise aus dem gummielastischen Material hergestellt. Dies ermöglicht ein elastisches Verformen der Öffnung. Typischerweise ist die Gummielastizität derart, dass ein Körper mit einem Durchmesser, der 10% größer ist als der Durchmesser der Öffnung zerstörungsfrei durch die Öffnung hindurchgeführt werden kann. Bevorzugt gilt das auch bei einem um 20% oder um 30% größeren Durchmesser. Die Öffnung mit dem Abstreifer ist insbesondere geringfügig kleiner als das zu beschichtende Implantat. So kann ein unkontrolliertes Ausfließen des Beschichtungsmittels verhindert werden.

Die Gummielastizität erlaubt zudem die Beschichtung von Implantaten mit einer entlang der Längsachse veränderlichen Erstreckung, z. B. einem variablen Durchmesser. Derartige Implantate werden in der Praxis häufig eingesetzt. Durch Dehnung des die Öffnung ausbildenden Materials wird eine definierte Beschichtung ermöglicht.

Ein längliches Implantat im Sinne der Erfindung ist ein Implantat, dessen Erstreckung in einer Haupterstreckungsrichtung um einen Faktor von wenigstens 3 größer ist als in allen Richtungen quer zur Haupterstreckungsrichtung. Ein Querschnitt des Implantats kann beispielsweise kreisförmig, elliptisch oder mehreckig sein. Mehreckige Querschnitte haben dabei mindestens drei Ecken. Das Implantat kann stabförmig sein.

In einer Ausgestaltung umfasst das Unterteil einen hohlen konischen Abschnitt. Insbesondere ist der konische Abschnitt teilweise oder vollständig aus dem gummielastischen Material hergestellt.

Die Vorrichtung ist demnach zweiteilig aufgebaut. Ein Oberteil wird durch den Vorratsbehälter definiert und das Unterteil umfasst den konischen Abschnitt. Während der bestimmungsgemäßen Verwendung befindet sich der Vorratsbehälter typischerweise oberhalb des konischen Abschnitts. Ein Beschichtungsmittel kann sich im Vorratsbehälter und/oder im konischen Abschnitt befinden. Das Implantat kann im Vorratsbehälter und/oder im konischen Abschnitt mit dem Beschichtungsmittel in Kontakt kommen. Der konische Abschnitt kann sich an die Form des Implantats anpassen und so eine besonders effektive Beschichtung ermöglichen.

Das Unterteil kann den Vorratsbehälter mit der Öffnung verbinden und/oder der Beschichtung des Implantats dienen. Das Unterteil und/oder der konische Abschnitt definiert typischerweise einen Hohlraum, in dem Beschichtungsmittel aufgenommen werden kann und/oder durch den das Implantat bewegt werden kann. Der Hohlraum des Vorratsbehälters ist mit dem Hohlraum des Unterteil bzw. des konischen Abschnitts verbunden. Insbesondere ist die Öffnung an der dem Vorratsbehälter abgewandten Seite des konischen Abschnitts angeordnet.

Der konische Abschnitt weist insbesondere eine konische Außenfläche auf. Die Innenfläche kann ebenso konisch geformt sein. Die Wandstärke kann konstant sein. Eine zentrale Achse des konischen Abschnitts verläuft insbesondere von einer größeren ersten Seite zu einer kleineren zweiten Seite. Der konische Abschnitt kann eine rotationssymmetrische, drehsymmetrische, spiegelsymmetrische und/oder langgezogene Grundform aufweisen. Die zentrale Achse kann mit der Rotationsachse, der Symmetrieachse und/oder einer mittleren Achse einer Symmetrieebene übereinstimmen. Der konische Abschnitt ist insbesondere länglich geformt. Die Längsachse des konischen Abschnitts kann demnach mit der zentralen Achse übereinstimmen. Der konische Abschnitt kann die Form eines hohlen Kegelstumpfs aufweisen. Die Öffnung befindet sich insbesondere an der kleineren zweiten Seite und/oder der dem Vorratsbehälter abgewandten Seite des konischen Abschnitts.

Ein Winkel zwischen einer Außenseite des konischen Abschnitts und der zentralen Achse des konischen Abschnitts ist typischerweise höchstens 45°, bevorzugt höchstens 35° und insbesondere höchstens 25°. Besonders bevorzugt ist der Winkel höchstens 20° und insbesondere höchstens 15°. Der Winkel ist typischerweise wenigstens 2°, insbesondere wenigstens 5°.

Bevorzugt ist das Unterteil mechanisch mit dem Vorratsbehälter verbunden oder verbindbar. Es kann eine manuelle und/oder reversible Verbindung vorgesehen sein. Die Verbindung ist insbesondere flüssigkeitsdicht.

Der konische Abschnitt ist insbesondere teilweise oder vollständig aus einem gummielastischen Material hergestellt. Dies ermöglicht ein elastisches Verformen.

In einer Ausführungsform ist das die Öffnung ausbildende Material, ein Material des Unterteils oder ein Material des konischen Abschnitts ein Material mit einer Shore-A-Härte von mindestens 30, bevorzugt mindestens 50. Insbesondere ist die Shore-A-Härte des Materials höchstens 80.

In einer Ausgestaltung weist eine größere erste Seite des konischen Abschnitts zum Vorratsbehälter hin und eine kleinere zweite Seite des konischen Abschnitts weist vom Vorratsbehälter weg. Insbesondere befindet sich die Öffnung an der zweiten Seite.

Der konische Abschnitt weist zwei Seiten auf, insbesondere an entgegengesetzten Positionen mit Bezug auf eine Längsachse und/oder eine zentrale Achse des konischen Abschnitts. Die größere Seite ist größer als die kleinere Seite. Die erste Seite ist insbesondere mit dem Vorratsbehälter verbunden oder verbindbar. Der Abstreifer kann an der zweiten Seite angeordnet sein. Der Abstreifer befindet sich insbesondere am vom Vorratsbehälter weg weisenden oder freien Ende des konischen Abschnitts.

Die erste Seite umfasst eine Öffnung, die mit dem Vorratsbehälter durchgängig verbunden ist. Die Erstreckung dieser Öffnung, beispielsweise der freie Durchmesser, entspricht mindestens der Quererstreckung, beispielsweise dem Außendurchmesser, des zu beschichtenden Implantats.

In einer Ausgestaltung weist der Vorratsbehälter einen ersten Verbindungsbereich auf und das Unterteil weist einen korrespondierenden zweiten Verbindungsbereich auf. Insbesondere sind der erste Verbindungsbereich und der zweite Verbindungsbereich mechanisch reversibel miteinander verbindbar.

Das Unterteil und das Oberteil sind demnach reversibel verbindbar. Reversibel meint, dass die Verbindung zerstörungsfrei lösbar ist. Insbesondere kann die Verbindung manuell hergestellt und/oder gelöst werden. Beispielsweise sind die Verbindungsbereiche als korrespondierende Gewinde, als korrespondierende Teile eines Bajonettverschlusses oder als korrespondierende Teile einer Steckverbindung ausgestaltet.

Dies ermöglicht ein einfaches Austauschen des konischen Abschnitts bzw. ein Montieren eines gewünschten konischen Abschnitts. Es können unterschiedliche konische Abschnitte für unterschiedlichen Implantate vorgesehen sein. Beispielsweise kann ein konischer Abschnitt für ein Implantat mit Kreisquerschnitt eine Öffnung mit einer kreisrunden Grundform aufweisen und/oder ein konischer Abschnitt für ein flaches Implantat eine Öffnung mit einer länglichen Grundform aufweisen. Die tatsächliche Form kann z. B. aufgrund von Abstandhaltern von der Grundform abweichen. An der dem Vorratsbehälter zugewandten Seite können die unterschiedlichen konischen Abschnitte dagegen gleich oder ähnlich geformt sein.

In einer Ausgestaltung weist die Öffnung eine kreisrunde oder schlitzförmige Grundform auf. Auf diese Weise können Marknägel mit kreisförmigen Querschnitt oder Osteosyntheseplatten mit flachem Querschnitt besonders gleichmäßig beschichtet werden. Die tatsächliche Form der Öffnung kann von der Grundform abweichen, etwa aufgrund der Abstandhalter.

In einer Ausgestaltung sind am Abstreifer Abstandhalter angeordnet, um einen Abstand zwischen dem Abstreifer und einem zu beschichtenden länglichen Implantat zu gewährleisten. Abstandhalter sorgen für einen definierten Abstand zwischen dem Abstreifer und dem Implantat, wenn das Implantat durch die Öffnung bewegt wird. Dies ermöglicht eine Beschichtung mit einer einheitlichen Schichtdicke.

Da die Abstandhalter das Implantat direkt kontaktieren, befindet sich an diesen Kontaktstellen dann weniger oder kein Beschichtungsmittel. Es bilden sich dann Längsstreifen ohne Beschichtung an der Oberfläche des Implantats. Es hat sich gezeigt, dass bei einigen Beschichtungsmitteln, beispielsweise dünnflüssigen Knochenzementen, das Beschichtungsmittel nach der Beschichtung auch in die nicht beschichteten Bereiche fließt und auf diese Weise eine einheitliche Beschichtung erreicht wird.

Insbesondere ragen die Abstandhalter radial nach innen. Bevorzugt sind die Abstandhalter so ausgebildet, dass der größte Teil der Oberfläche des Implantats beschichtet wird. Der Anteil der Oberfläche des Implantats, an der das Beschichtungsmittel aufgetragen wird, ist also mindestens so groß und bevorzugt größer als der Anteil der Fläche, der mit den Abstandhaltern in Kontakt steht. Es hat sich herausgestellt, dass eine Beschichtung von mindestens 50% der Oberfläche ausreichend ist, um eine effektive Versorgung mit dem Wirkstoff sicherzustellen. Bevorzugt sind die Abstandhalter in gleichen Abständen angeordnet, um die Beschichtung gleichmäßig aufzutragen.

Die Abstandhalter sind insbesondere an einer Innenfläche des konischen Abschnitts und/oder des Abstreifers angeordnet. Auf diese Weise kann beim Abstreifen des Beschichtungsmittels ein Abstand des Abstreifers von dem Implantat gewährleistet werden.

Insbesondere sind die Abstandhalter in Längsrichtung verteilt angeordnet, bevorzugt in gleichmäßigen Abständen. Die Abstandhalter können z. B. stiftförmig oder stegförmig ausgebildet sein.

Die Abstandhalter sind insbesondere am konischen Bereich befestigt oder angeformt. Insbesondere sind die Abstandhalter nicht aus dem gummielastischen Material des konischen Abschnitts hergestellt, sondern aus einem Material mit einer höheren Steifigkeit.

Im Falle einer schlitzförmigen Öffnung kann vorgesehen sein, dass lediglich die im Querschnitt betrachtet langen Seiten beschichtet werden. An den Schmalseiten sind dann keine Abstandhalter angeordnet. Es kann alternativ oder ergänzend vorgesehen sein, nur eine Seite zu beschichten. Auf der nicht zu beschichtenden Seite sind dann keine Abstandhalter angeordnet, sodass das Beschichtungsmittel vollständig oder nahezu vollständig abgestreift wird.

Die Abstandhalter sind typischerweise nicht aus dem die Öffnung ausbildenden gummielastischen Material hergestellt. Die Abstandhalter sind typischerweise aus einem härteren und/oder festeren Material hergestellt. Ein gummielastisches Ausweichen der Abstandhalter ist nicht gewünscht. In einer Ausführungsform sind die Abstandhalter aus einem Kunststoff, bevorzugt einem thermoplastischen Kunststoff, hergestellt.

Die Abstandhalter können an dem konischen Abschnitt befestigt sein. Die Abstandhalter können alternativ oder ergänzend an einer separaten Struktur, insbesondere des Unterteils, befestigt sein. In diesem Fall ist es möglich, dass die Abstandhalter am konischen Abschnitt anliegen.

In einer Ausgestaltung sind die Abstandhalter in Umfangsrichtung gleichmäßig verteilt angeordnet. Dies erlaubt ein gleichmäßiges Auftragen der Beschichtung. Insbesondere sind in Umfangsrichtung wenigstens drei Abstandhalter vorhanden, bevorzugt wenigstens vier oder wenigstens fünf Abstandhalter. In einer Ausführungsform sind in Umfangsrichtung sechs oder mehr Abstandhalter angeordnet.

In einer Ausgestaltung sind die Abstandhalter stegförmig ausgestaltet. Stegförmig meint eine Form, bei der eine erste Erstreckungsrichtung um ein Vielfaches länger ist als die zweite und die dritte Erstreckungsrichtung, die beide senkrecht zur ersten und zueinander ausgerichtet sind. Beispielsweise kann die erste Erstreckungsrichtung um einen Faktor von wenigstens 3, bevorzugt wenigstens 5 und besonders bevorzugt wenigstens 8 oder 10 größer sein als die zweite und/oder dritte Erstreckungsrichtung. Die stegförmigen Abstandhalter können am konischen Abschnitt angeordnet sein.

Bevorzugt verlaufen die stegförmigen Abstandhalter in Längsrichtung der Vorrichtung und/oder des konischen Abschnitts.

In einer Ausgestaltung verlaufen die Abstandhalter in einem Winkel von weniger als 30° zu einer zentralen Achse der Vorrichtung. Durch die konische Form des konischen Abschnitts verlaufen die Abstandhalter allerdings nicht exakt parallel zur zentralen Achse oder zur Außenwand des konischen Abschnitts, sondern, wie auch die Außenseite des konischen Abschnitts, in einem Winkel dazu. Der Winkel zwischen einer Längsachse eines stegförmigen Abstandhalters und der zentralen Achse des konischen Abschnitts kann grundsätzlich höchstens 45°, bevorzugt höchstens 35° und insbesondere höchstens 25° betragen. Besonders bevorzugt ist der Winkel höchstens 20° und insbesondere höchstens 15°. Der Winkel beträgt typischerweise wenigstens 2°, insbesondere wenigstens 5°.

Durch die längliche Form können die Abstandhalter nicht in Längsrichtung ausweichen. Dies gewährleistet das Halten des Abstands, wenn das Implantat entlang der zentralen Achse durch die Vorrichtung bewegt wird, auf besonders sichere Weise. Die stegförmigen Abstandhalter erlauben auch im Falle von Dehnung des die Öffnung ausbildenden Materials infolge eines veränderlichen Querschnitts des Implantats die Herstellung einer Beschichtung mit konstanter Dicke.

In einer Ausführungsform sind die stegförmigen Abstandhalter so geformt, dass sie maximal 35% des Umfangs des Implantats berühren, vorzugsweise maximal 25% und besonders bevorzugt maximal 15%.

In einer Ausführungsform weisen die stegförmigen Abstandhalter entlang ihrer Haupterstreckungsrichtung eine Länge von mindestens 3 mm, bevorzugt mindestens 5 mm und/oder höchstens 20 mm, bevorzugt höchstens 15 mm auf. In einer Ausführungsform weisen die stegförmigen Abstandhalter in einer Richtung quer zu ihrer Haupterstreckungsrichtung eine Breite von wenigstens 0,5 mm, insbesondere wenigstens 1,0 mm und/oder höchstens 5,0 mm, insbesondere höchstens 3,0 mm auf. Dies kann für beide Richtungen quer zu der Haupterstreckungsrichtung gelten. In den beiden Richtungen können gleiche oder unterschiedliche Erstreckungen vorliegen. Die Erstreckung der Abstandhalter in radialer Richtung definiert dabei die Schichtdicke. Insbesondere weisen alle Abstandhalter zumindest in radialer Richtung und bevorzugt auch in allen anderen Richtungen die gleichen Erstreckungen auf.

In einer Ausgestaltung weisen die stegförmigen Abstandhalter einen Querschnitt mit einer Anlageseite und einer freien Seite auf. Insbesondere ist eine erste Breite der Abstandhalter an der Anlageseite zumindest bereichsweise größer als eine parallel zur ersten Breite gemessene zweite Breite der Abstandhalter an der freien Seite. Die Anlageseite liegt am konischen Abschnitt an und kann daran befestigt sein. Die freie Seite ragt frei nach innen und dient der Kontaktierung des Implantats.

Mit anderen Worten weisen die Abstandhalter eine senkrecht zur Längserstreckung gemessene Quererstreckung auf, die im Bereich einer Innenwandung des konischen Abschnitts zumindest bereichsweise breiter ist als im Bereich des freien Endes.

Auf diese Weise wird die Kontaktfläche zwischen dem Implantat und dem Abstandhalter minimiert. Der nicht beschichtete Anteil der Oberfläche des Implantats wird dadurch verringert. So kann eine besonders gleichmäßige Beschichtung erreicht werden.

In einer Ausgestaltung weisen die stegförmigen Abstandhalter einen dreieckigen, trapezförmigen, rechteckigen oder halbkugelförmigen Querschnitt auf. Eine solche Form des Abstandhalters minimiert den nicht beschichteten Bereich des Implantats, kann einfach hergestellt werden und ist besonders formstabil. Ecken des Querschnitts können gerundet sein.

In einer Ausgestaltung ist ein zum Vorratsbehälter hin weisendes Ende der stegförmigen Abstandhalter gerundet ausgeführt. Auf diese Weise kann ein Einführen des Implantats vereinfacht werden und es kann eine besonders reproduzierbare Beschichtung gewährleistet werden.

In einer Ausgestaltung ist zumindest eine dem Unterteil und/oder dem konischen Abschnitt abgewandte Seite des Vorratsbehälters als Zylinder ausgebildet. Insbesondere weist die Vorrichtung ferner einen dem Zylinder korrespondierenden Kolben zum Pressen des Beschichtungsmittels auf. Der Kolben ist typischerweise ein separates Bauteil.

Zylinder und Kolben sind korrespondierend geformt. Der Kolben kann typischerweise manuell in dem Zylinder bewegt werden, um das Beschichtungsmittel aus dem Vorratsbehälter in Richtung des konischen Abschnitts und/oder der Öffnung zu pressen. Dies ist insbesondere im Falle viskoser oder hochviskoser Knochenzemente vorteilhaft, die nur langsam fließen. In diesem Fall kann es vorkommen, dass der an dem Implantat anhaftende Knochenzement rascher nach unten aus dem konischen Abschnitt ausgetragen wird als Knochenzement aus dem Vorratsbehälter nachfließt. Durch die Möglichkeit, den Knochenzement nach unten zu drücken, können Fehlstellen der Beschichtung durch fehlenden Knochenzement verhindert werden und es kann eine rasche und gleichmäßige Beschichtung gewährleistet werden.

Es kann ein Teil des Vorratsbehälters oder der gesamte Vorratsbehälter als Zylinder ausgestaltet sein. Der Zylinder muss kein Kreiszylinder sein, sondern kann eine beliebige Querschnittsfläche aufweisen. Ein Kreiszylinder ist aber besonders einfach herstellbar. Der Kolben und der Zylinder weisen insbesondere eine gemeinsame zentrale Achse auf, entlang welcher die relative Bewegung erfolgt. Der Kolben kann einen U-förmigen Querschnitt aufweisen. Alternativ kann der Kolben als beispielsweise konvexer Hohlkörper ausgebildet sein.

In einer Ausführungsform weist der Kolben kann eine insbesondere mittige Öffnung auf, durch die das Implantat durchgeführt werden kann. Die Öffnung kann mit einer insbesondere gummielastischen Abdeckung teilweise oder ganz verschlossen sein. Da der Kolben in der Regel bei hochviskosem Knochenzement verwendet wird, kann er trotz der Öffnung Knochenzement nach unten drücken. Im Bereich der Öffnung kann ein Abstreifer angeordnet sein.

Der Vorratsbehälter, das Unterteil und/oder der konische Abschnitt sind insbesondere aus Kunststoff und/oder Gummi hergestellt. Bevorzugt sind der Vorratsbehälter und das Unterteil mit Ausnahme des konischen Abschnitts aus Kunststoff hergestellt und/oder der konische Abschnitt aus einem gummielastischen Material wie z. B. Gummi.

Ein weiterer Aspekt der Erfindung ist ein System, insbesondere zur Beschichtung eines länglichen Implantats. Das System umfasst eine erfindungsgemäße Vorrichtung. Das System umfasst ferner ein zusätzliches Unterteil mit einer Öffnung, wobei das die Öffnung ausbildende Material gummielastisch ist und als Abstreifer dient. Das zusätzliche Unterteil weist einen zweiten Verbindungsbereich auf, der mit dem ersten Verbindungsbereich der Vorrichtung korrespondiert und mechanisch reversibel mit diesem verbunden werden kann.

So kann vor der Beschichtung durch Wahl und Verbinden eines gewünschten konischen Abschnitts eine gewünschte Schichtdicke eingestellt werden. Das System kann auch als Kit bezeichnet werden. Die Komponenten des Systems sind insbesondere zusammen verpackt, so dass intraoperativ der geeignete konische Abschnitt, also das Unterteil der Vorrichtung, gewählt und verwendet werden kann. Anders ausgedrückt umfasst das System ein Oberteil und mindestens zwei Unterteile. Es können mehrere zusätzliche Unterteile vorhanden sein, wobei jede Öffnung jedes Unterteils eine unterschiedliche Form und/oder Größe aufweist.

Alle Merkmale, Vorteile und Ausgestaltungen der eingangs beschriebenen Vorrichtung, der Verwendung und des Verfahrens können auch für das System gelten und umgekehrt.

In einer Ausführungsform umfasst das System ferner Komponenten zur Herstellung von Knochenzement. Die Komponenten sind insbesondere derart, dass ohne Zuführung weiterer Materialien Knochenzement hergestellt werden kann. Beispielsweise sind als Komponenten Polymethylmethacrylat-Knochenzementpulver und Monomerflüssigkeit vorhanden. Insbesondere sind die Komponenten in jeweils separaten Packmitteln enthalten. Etwaige Wirkstoffe müssen typischerweise hinzugefügt werden.

In einer Ausführungsform umfasst das System ferner eine Mischvorrichtung zum Anmischen von Knochenzement. Die Mischvorrichtung kann eine Mischschüssel und ein oder mehrere Mischwerkzeuge wie z. B. einen oder mehrere Spatel umfassen. Alternativ oder ergänzend kann die Mischvorrichtung ein Mischsystem umfassen, beispielsweise mit einer Kartusche mit integrierter oder montierter Mischstange.

Ein weiterer Aspekt der Erfindung ist eine Verwendung einer erfindungsgemäßen Vorrichtung zur Beschichtung eines länglichen Implantats. Alle Merkmale, Vorteile und Ausgestaltungen der eingangs beschriebenen Vorrichtung sowie des Systems und des Verfahrens können auch für die Verwendung gelten und umgekehrt.

Das Implantat ist insbesondere ein chirurgisches Implantat wie z. B. ein beispielsweise verriegelter oder nicht verriegelter Marknagel (intramedullärer Nagel) oder eine Osteosyntheseplatte. Das Implantat kann z. B. aus Titan, Titanlegierungen, Stahl, einem Kompositmaterial und/oder einem oder mehreren Kunststoffen hergestellt sein oder eines oder mehrere dieser Materialien enthalten. Das zu beschichtende Implantat kann grundsätzlich auch ein beliebiges anderes längliches Implantat wie z. B. eine implantierbare Elektrode, eine schlauchförmige Gefäßprothese oder ein Kunststoffschlauch sein. Grundsätzlich können auch andere längliche Gegenstände beschichtet werden.

Insbesondere Beschichtung mit einem pastösen Beschichtungsmittel, z. B. Knochenzement, wie z. B. Polymethylmethacrylat-Knochenzement. Polymethylmethacrylat-Knochenzement wird insbesondere durch Vermischung von Polymethylmethacrylat-Knochenzementpulver und Methylmethacrylat-Monomerflüssigkeit hergestellt und härtet durch radikalische Polymerisation des Methylmethacrylats selbstständig innerhalb weniger Minuten aus. Insbesondere enthält die Beschichtung mindestens einen darin suspendierten oder gelösten pharmazeutischen Wirkstoff.

Alternativ oder ergänzend kann ein Polymer bzw. eine Polymerlösung verwendet werden. Hierzu eignen sich zum Beispiel Polylactid, Polymethylmethacrylat, Polyvinylacetat und/oder Polyvinylchlorid, die in flüchtigen Lösungsmitteln, wie zum Beispiel Aceton und/oder Chloroform, gelöst sind. Das flüchtige Lösungsmittel kann nach dem Beschichten aus der Polymerlösung verdampfen und einen haftenden Polymerfilm auf der Oberfläche des Implantats hinterlassen.

Insbesondere ist in dem Beschichtungsmittel mindestens ein pharmazeutischer Wirkstoff enthalten, wobei der mindestens eine pharmazeutische Wirkstoff bevorzugt ein Antiinfektivum ist wie z. B. Gentamicin, Tobramycin, Amikacin, Vancomycin, Dalbavancin, Teicoplanin, Daptomycin, Clindamycin, Meropenem, Colistin, Ampicillin, Amoxicillin, Ofloxacin, Levofloxacin, Moxifloxacin, Ciprofloxacin, Amphotericin B, Micafungin, Fluconazol oder eine beliebige Mischung aus mindestens zwei der Genannten. Neben ihrer eigentlichen, in der Regel mechanischen Funktion, sind die beschichteten Implantate dann auch zur lokalen Freisetzung eines Wirkstoffs geeignet.

Ein weiterer Aspekt der Erfindung ist ein Verfahren zur Beschichtung eines länglichen Implantats unter Verwendung einer Vorrichtung. Das Verfahren umfasst Bereitstellen einer Vorrichtung mit einem Vorratsbehälter, in dem ein Beschichtungsmittel aufgenommen ist. Das Verfahren umfasst ferner Bewegen eines länglichen Implantats durch das Beschichtungsmittel sowie durch eine Öffnung in einem Unterteil der Vorrichtung. Überschüssiges Beschichtungsmittel wird durch einen Abstreifer der Vorrichtung von dem Implantat abgestreift.

Die Bewegung erfolgt insbesondere in eine Richtung vom Vorratsbehälter zum konischen Abschnitt. Das Bewegen des Implantats durch das Beschichtungsmittel kann beispielsweise in dem Vorratsbehälter und/oder in dem Unterteil erfolgen. Das Abstreifen erfolgt insbesondere beim Verlassen des Unterteils, bevorzugt durch eine Öffnung, die den Abstreifer umfasst oder ausbildet. Das Implantat kann länger oder kürzer sein als die Vorrichtung. Es kann sich ein noch unbeschichteter Teil des Implantats oberhalb des Beschichtungsmittels befinden, während überschüssiges Beschichtungsmittel von einem bereits beschichteten Teil des Implantats abgestreift wird. Das Implantat kann die gesamte Vorrichtung durchlaufen. Insbesondere ist die Vorrichtung eine erfindungsgemäße Vorrichtung.

Die Bewegung ist grundsätzlich eine Relativbewegung. Es ist unerheblich, ob das Implantat, die Vorrichtung oder beide Komponenten bewegt werden. Der Einfachheit halber wird hier lediglich von einem "Bewegen" oder "Bewegen des Implantats" gesprochen. Es sind immer alle oben genannten Möglichkeiten der Relativbewegung gemeint.

Weist das Implantat einen veränderlichen Querschnitt auf, wird insbesondere eine dünnere Seite des Implantats zuerst beschichtet. Das Implantat wird dann mit der dünneren Seite zuerst nach unten in die Vorrichtung bewegt.

Insbesondere erfolgt zunächst ein Bewegen des Implantats derart, dass ein erstes Ende des Implantats sich im Bereich einer insbesondere unten liegenden, zweiten Öffnung des konischen Abschnitts befindet. Das erste Ende kann durch die zweite Öffnung hindurchgesteckt werden. Anschließend wird insbesondere das Beschichtungsmittel in den Vorratsbehälter und/oder den konischen Abschnitt eingefüllt. Insbesondere ist dabei die Vorrichtung so ausgerichtet, dass sich der Vorratsbehälter mittig oberhalb des konischen Abschnitts befindet. Wird diese Reihenfolge eingehalten, wird effektiv verhindert, dass Beschichtungsmittel in einen inneren Hohlraum des Implantats eindringt. Der Hintergrund ist der, dass Marknägel und andere Implantate als an den Enden offene Hohlkörper ausgebildet sein können. Ein Eindringen von Beschichtungsmittel in den Hohlraum ist in diesen Fällen zumeist nicht gewünscht.

Sobald das Implantat sich im Bereich der Öffnung befindet, kann das die Öffnung ausbildende Material durch den Kontakt mit dem Implantat gedehnt werden.

Schwerkraftbedingt kann das Beschichtungsmittel nach unten in den konischen Abschnitt sinken. Dort kann das Implantat benetzt werden. Das Beschichtungsmittel haftet dann an der Oberfläche des Implantats.

Das Implantat kann relativ zur Vorrichtung nach unten bewegt werden, etwa durch Drücken am oberen Ende und/oder Ziehen am unteren Ende. Bevorzugt wird das gesamte Implantat durch die Vorrichtung bewegt, so dass jeder Bereich des Implantats einmal mit dem Abstreifer in Kontakt gelangt ist. Währenddessen kann Beschichtungsmittel aus dem Vorratsbehälter nach unten in den konischen Abschnitt nachfließen. Dies kann durch Pressen eines Kolbens unterstützt werden.

Abstandhalter wie z. B. stegförmige Abstandhalter können das Implantat kontaktieren und so einen Abstand zwischen dem Abstreifer und der zu Oberfläche des Implantats halten. So kann zumindest in den Bereichen zwischen den Abstandhaltern eine definierte, gleichmäßige Schichtdicke erzielt werden, die der radialen Erstreckung der Abstandhalter entspricht. Überschüssiges Beschichtungsmittel wird abgestreift und verbleibt im konischen Abschnitt.

Insbesondere erfolgt anschließend eine Nachbereitung des Beschichtens zur Herstellung der endgültigen, festen Beschichtung. Dies kann beispielsweise erfolgen durch ein Aushärten des Beschichtungsmittels oder ein Verdunsten und/oder Verdampfen von Lösungsmittel.

In einer Ausführungsform erfolgt ein Entfernen von Beschichtungsmittel aus Öffnungen des Implantats, beispielsweise durch Aufstecken mittels eines Stifts oder Stößels. Dies erfolgt bevorzugt vor der Nachbereitung.

Nachfolgend werden Ausführungsbeispiele der Erfindung auch anhand von Figuren näher erläutert. Merkmale der Ausführungsbeispiele können einzeln oder in einer

Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine perspektivische Schnittzeichnung einer Vorrichtung,
- Figur 2:: zwei Schnittzeichnungen einer Vorrichtung,
- Figur 3:: eine Schnittzeichnungen einer Vorrichtung bei der Verwendung,
- Figur 4:: eine Schnittzeichnungen einer Vorrichtung bei der Verwendung,
- Figur 5:: ein beschichtetes Implantat,
- Figur 6:: eine Explosionszeichnung einer Vorrichtung,
- Figur 7:: eine Schnittzeichnungen einer Vorrichtung bei der Verwendung,
- Figuren 8 und 9:: Querschnitte beschichteter Implantate, sowie
- Figur 10:: eine Schnittzeichnung eines Abstandhalters.

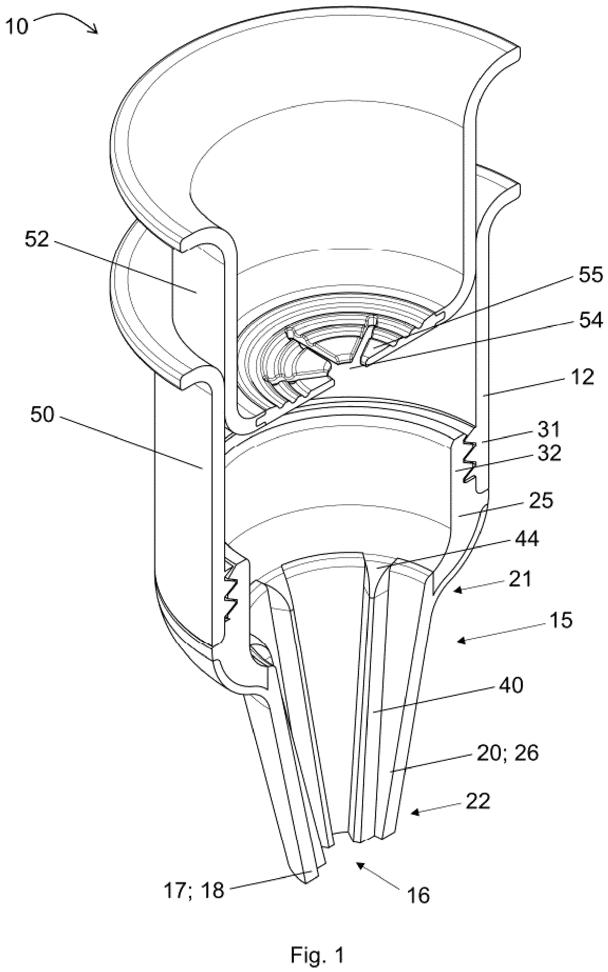

Figur 1 zeigt eine erfindungsgemäße Vorrichtung 10 zum Beschichten länglicher Implantate. Die Vorrichtung 10 umfasst einen hier mittig dargestellten Vorratsbehälter 12 zur Aufnahme eines Beschichtungsmittels. Der Vorratsbehälter 12 hat eine kreiszylinderförmige bzw. rotationssymmetrische Grundform und ist an der Oberseite sowie an der Unterseite offen. An der Unterseite umfasst der Vorratsbehälter 12 einen ersten Verbindungsbereich 31 in Form eines Innengewindes.

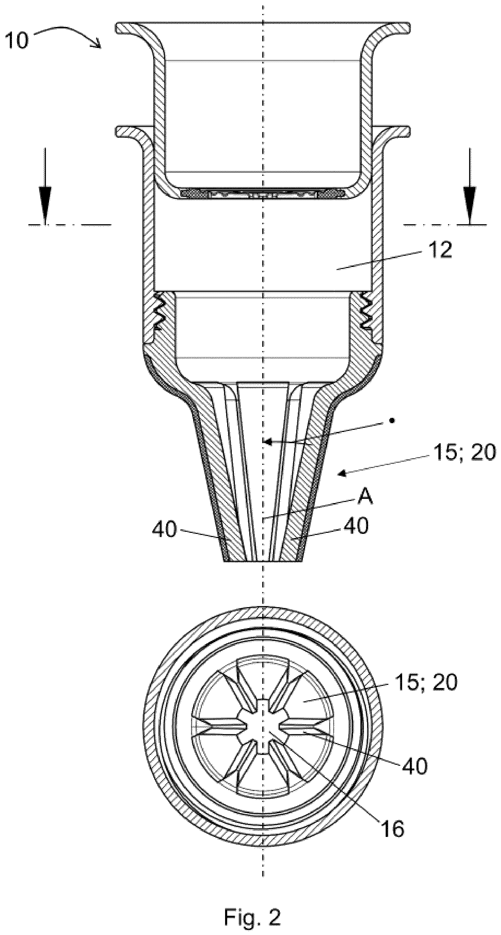

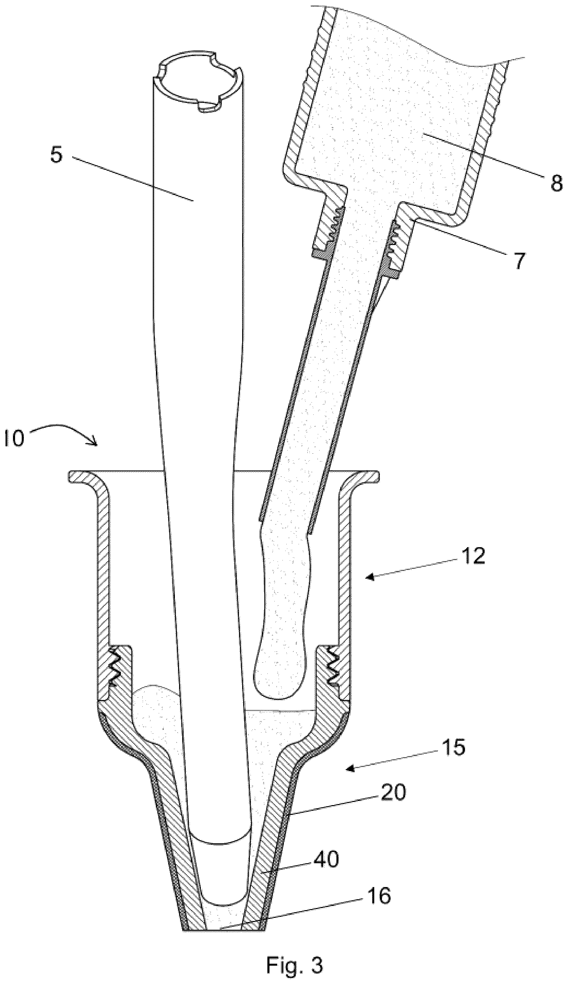

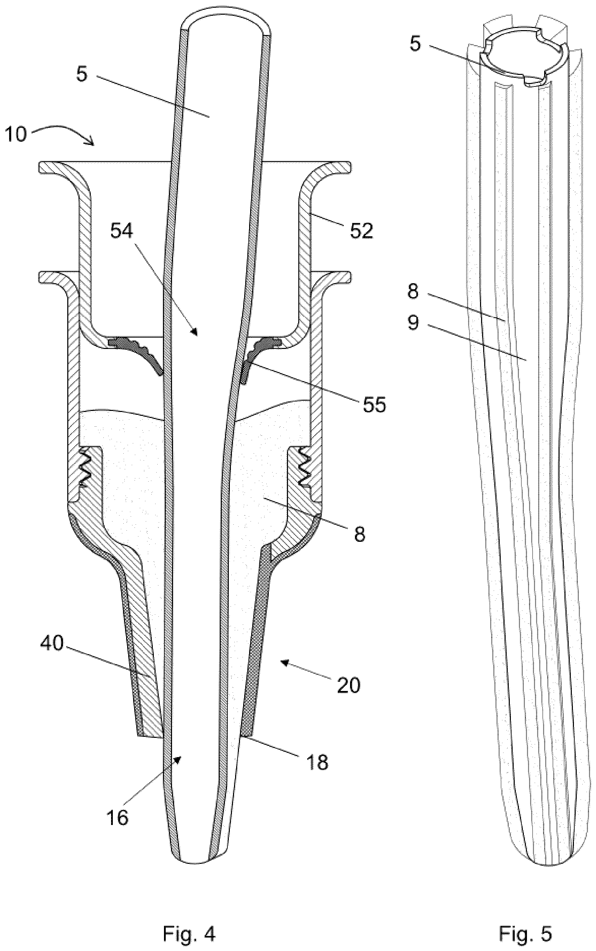

Unterhalb des Vorratsbehälters 12 befindet sich das Unterteil 15, welches hier aus zwei insbesondere nicht zerstörungsfrei voneinander lösbaren Komponenten 25, 26 zusammengesetzt ist. Eine etwa ringförmige obere Komponente 25 umfasst einen zweiten Verbindungsteil 32 in Form eines Außengewindes, das in das Innengewinde des Vorratsbehälters 12 geschraubt werden kann. die obere Komponente 25 kann aus vergleichsweise steifem Kunststoff hergestellt sein. Eine untere Komponente 26 des Unterteils 15 ist als hohler konischer Abschnitt 20 ausgestaltet, der eine Grundform eines Kegelstumpfs aufweist. Die untere Komponente 26 kann aus gummielastischen Material hergestellt sein. Die größere erste Seite 21 des konischen Abschnitts 20 ist mit der oberen Komponente 25 verbunden. Die kleinere zweite Seite 22 des konischen Abschnitts 20 weist nach unten und bildet die Öffnung 16 der Vorrichtung aus. Die Öffnung 16 weist eine kreisrunde Grundform auf. Die exakte Form der Öffnung 16 weicht aufgrund der unten beschriebenen Abstandhalter 40 von der Kreisform ab. Das die Öffnung 16 ausbildende Material 17 ist gummielastisch und dient als Abstreifer 18 zum Abstreifen. Insbesondere ist die gesamte untere Komponente 26 und/oder der gesamte konische Abschnitt 20 gummielastisch. Die Verwendung der Vorrichtung 10 ist in den Figuren 3 und 4 dargestellt. Die obere Komponente 25 kann die unten beschriebenen Abstandhalter 40 umfassen, wie in Figur 2 dargestellt.

Im Unterteil 15, nämlich im konischen Abschnitt 20, sind Abstandhalter 40 angeordnet, um einen definierten Abstand zwischen der Oberfläche des Implantats und dem Abstreifer 18 zu gewährleisten. Die Abstandhalter 40 sind als im wesentlichen vertikale bzw. entlang der zentralen Achse A bzw. Längsachse der Vorrichtung 10 und/oder des konischen Abschnitts 20 ausgerichtete Stege ausgestaltet. Dies verhindert ein Ausweichen der Abstandhalter 40 in vertikaler Richtung, die auch als z-Richtung bezeichnet wird.

Das obere, zum Vorratsbehälter 12 hin weisende Ende 44 der Abstandhalter ist gerundet ausgeführt. So kann ein ungewünschtes Blockieren des Implantats bei der Abwärtsbewegung des Implantats verhindert werden.

Die hier gezeigte Ausführungsform zeigt drei Abstandhalter 40 in einer Hälfte der Vorrichtung 10, also insgesamt sechs Abstandhalter 40. Die Abstandhalter 40 sind in Umfangsrichtung gleichmäßig verteilt.

Die Vorrichtung umfasst ferner optional einen Kolben 52. In diesem Fall ist die obere Seite des Vorratsbehälters 12 zylinderförmig, hier kreiszylinderförmig. Der Kolben 52 passt genau in die obere Seite des Vorratsbehälters 12 und dient dazu, ein hochviskoses Beschichtungsmittel nach unten in den konischen Abschnitt 20 pressen. Der Kolben 52 umfasst eine Öffnung 54, die von einer gummielastischen, insbesondere sternförmig geschlitzten Abdeckung 55 zumindest teilweise verschlossen ist.

Figur 2 zeigt oben einen Längsschnitt einer Vorrichtung 10 und unten einen Querschnitt mit Blickrichtung wie im Längsschnitt eingezeichnet. Die Vorrichtung 10 kann der Vorrichtung 10 aus Figur 1 ganz oder teilweise entsprechen. Der Schnitt verläuft im Bereich des konischen Abschnitts 20 beidseitig durch jeweilige Abstandhalter 40. Der Winkel α zwischen einer Längsachse eines stegförmigen Abstandhalters 40 und einer hier vertikal verlaufenden zentralen Achse A der Vorrichtung 10 entspricht dem Winkel der inneren Oberfläche des konischen Abschnitts 20 zur zentralen Achse A der Vorrichtung.

In der unteren Darstellung ist erkennbar, dass die eine kreisförmige Grundform aufweisende Öffnung 16 aufgrund der Abstandhalter 40 sternförmig ist.

Figur 3 zeigt die Verwendung der Vorrichtung 10. Ein Implantat 5, insbesondere ein Marknagel, ist etwa vertikal in der Vorrichtung angeordnet. Ein Beschichtungsmittel 8 wie z. B. mit einem Wirkstoff versehender Knochenzement wird von oben in den Vorratsbehälter 12 und/oder das Unterteil 15 gegeben, beispielsweise mittels einer Spritze 7. Das untere Ende des Implantats 5 liegt zu dieser Zeit bereits an den Abstandhaltern 40 und/oder an der inneren Oberfläche des konischen Abschnitts 20 an oder ist bereits ein Stück weit durch die Öffnung 16 hindurchgeschoben. Auf diese Weise wird verhindert, dass Beschichtungsmittel 8 in den Innenraum des hohlen Implantats 5 gelangt.

Figur 4 zeigt einen weiteren Schritt bei der Verwendung der Vorrichtung 10. Der Schnitt verläuft in dieser Darstellung im Bereich des konischen Abschnitts 20 auf der linken Seite durch einen Abstandhalter 40 und auf der rechten Seite durch einen Bereich zwischen zwei Abstandhaltern, in dem kein Abstandhalter vorhanden ist. Das Implantat 5 ist weiter nach unten bewegt worden, und zwar etwa mittig durch die Vorrichtung. Es zeigt sich, dass die Wandung der Öffnung 16 auf der rechten Seite als Abstreifer 18 wirkt und überschüssiges Beschichtungsmittel 8 abstreift und zurückhält und dadurch eine konstante Schichtdicke des Beschichtungsmittels 8 auf dem Implantat 5 einstellt. Auf der rechten Seite dagegen kontaktiert der Abstandhalter 40 das Implantat 5 und verhindert so lokal eine Anlagerung von Beschichtungsmittel 8.

Es ist zudem gezeigt, dass das Implantat einen nach oben hin zunehmenden Durchmesser aufweist. Aufgrund der Gummielastizität des konischen Abschnitts 20 kann sich die Öffnung 16 weiten, so dass auch in diesem Fall eine konstante Schichtdicke erreicht werden kann.

Zusätzlich kann, wie ebenfalls in Figur 4 gezeigt, ein Kolben 52 verwendet werden, um das Beschichtungsmittel 8 nach unten zu pressen. Es ist dargestellt, wie das Implantat 5 durch die Öffnung 54 des Kolbens 52 hindurchgeführt ist. Dabei wird die flexible Abdeckung 55 der Öffnung 54 ausgelenkt.

Ein beschichtetes Implantat 5 ist beispielhaft in Figur 5 dargestellt. Das Implantat weist entlang seiner Längsachse verlaufende Streifen des Beschichtungsmittels 8 auf, die durch dazwischen liegenden Lücken 9 voneinander beabstandet sind. Die Lücken entsprechen den Positionen, in denen der Abstandhalter das Implantat 5 kontaktiert hat.

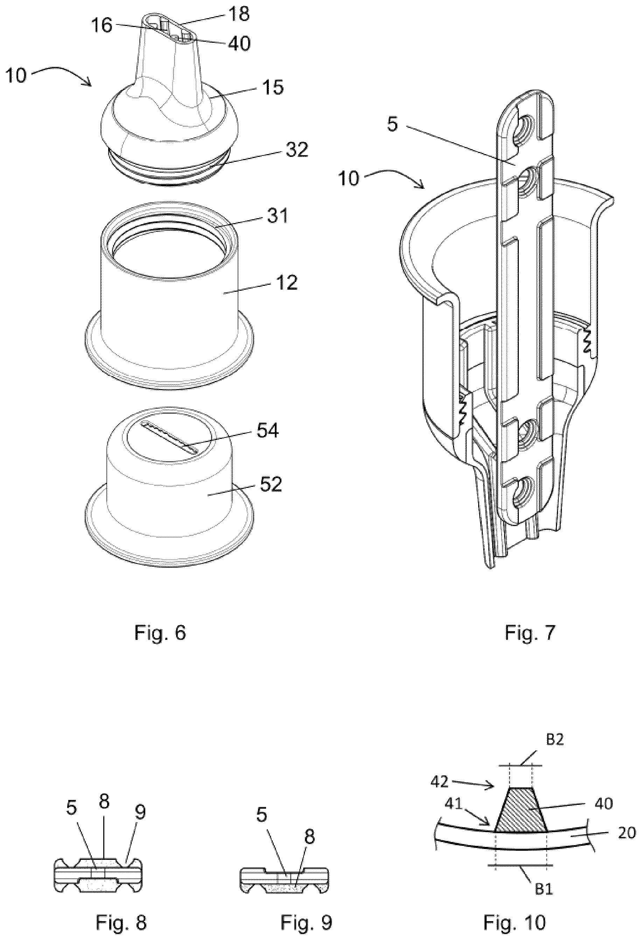

Eine weitere Ausführungsform einer Vorrichtung ist in Figur 6 anhand einer umgedrehten Explosionszeichnung dargestellt. Das hier oben gezeigte Unterteil 15 weist eine schlitzförmige Öffnung 16 auf, in der an den zwei langen Seiten jeweils zwei stegförmige Abstandhalter 40 sichtbar sind. Auch der konische Abschnitt ist schlitzförmig bzw. hat einen länglichen Querschnitt. Diese Vorrichtung dient, wie in Figur 7 beispielhaft anhand einer Osteosyntheseplatte gezeigt, dem Beschichten flacher Implantate 5. Auch die Öffnung 54 im optionalen Kolben 52 kann dann schlitzförmig ausgeführt sein. Grundsätzlich ist die Grundform der Öffnung dem äußeren Querschnitt des Implantats angepasst und kann beliebig gewählt sein.

Die Figuren 8 und 9 zeigen beschichtete Implantate 5 im Querschnitt. Beispielhaft für flache Implantate 5 sind Osteosyntheseplatten gezeigt. Üblicherweise werden nur die im Querschnitt gesehen langen Seiten beschichtet. Sind die Abstandhalter wie in Figur 6 beidseitig angeordnet, entsteht das in Figur 8 gezeigte Muster mit beidseitig angeordnetem Beschichtungsmittel 8 und dazwischen befindlichen Lücken 9. Sind dagegen nur einseitig Abstandhalter angeordnet, entsteht das in Figur 9 gezeigte Muster. Hierbei ist nur an einer Seite Beschichtungsmittel 8 angeordnet, mit jeweils dazwischen befindlichen Lücken 9, und auf der anderen Seite ist das gesamte Beschichtungsmittel durch den Abstreifer abgestreift und zurückgehalten worden.

Figur 10 zeigt einen Querschnitt durch einen stegförmigen Abstandhalter 40 mit Blickrichtung entlang einer inneren Oberfläche des konischen Abschnitts 20. Der Abstandhalter weist hier beispielhaft eine Trapezform auf. Die Anlageseite 41 des Abstandhalters 40 liegt an der inneren Oberfläche des konischen Abschnitts 20 an und die freie Seite 42 ragt zur Kontaktierung des Implantats frei nach radial innen. Die Breite B2 der freien Seite 42 ist geringer als die parallel dazu gemessene Breite B1 der Anlageseite 41.

**Bezugszeichenliste**

| | |
|---|---|
| Implantat | 5 |
| Spritze | 7 |
| Beschichtungsmittel | 8 |
| Lücke | 9 |
| Vorrichtung | 10 |
| Vorratsbehälter | 12 |
| Unterteil | 15 |
| Öffnung | 16 |
| Material | 17 |
| Abstreifer | 18 |
| konischer Abschnitt | 20 |
| erste Seite | 21 |
| zweite Seite | 22 |
| obere Komponente | 25 |
| untere Komponente | 26 |
| erster Verbindungsbereich | 31 |
| zweiter Verbindungsbereich | 32 |
| Abstandhalter | 40 |
| Anlageseite | 41 |
| freie Seite | 42 |
| Ende | 44 |
| erste Breite (BS) | B1 |
| zweite Breite (FS) | B2 |
| Zylinder | 50 |
| Kolben | 52 |
| Öffnung | 54 |
| Abdeckung | 55 |
| Zentrale Achse | A |
| Winkel | α |

## Patentansprüche

1. Vorrichtung (10) zur Beschichtung eines länglichen Implantats (5), umfassend einen Vorratsbehälter (12) zur Aufnahme eines Beschichtungsmittels (8) sowie ein mit dem Vorratsbehälter (12) verbundenes oder verbindbares Unterteil (15) mit einer Öffnung (16), wobei ein die Öffnung (16) ausbildendes Material (17) gummielastisch ist und als Abstreifer (18) zum Abstreifen von überschüssigem Beschichtungsmittel (8) dient.

2. Vorrichtung (10) nach dem vorhergehenden Anspruch, wobei das Unterteil (15) einen hohlen konischen Abschnitt (20) umfasst, wobei der konische Abschnitt (20) insbesondere aus dem gummielastischen Material hergestellt ist.

3. Vorrichtung (10) nach dem vorhergehenden Anspruch, wobei eine größere erste Seite (21) des konischen Abschnitts (20) zum Vorratsbehälter (12) hin weist und eine kleinere zweite Seite (22) des konischen Abschnitts (20) vom Vorratsbehälter (12) weg weist, wobei sich die Öffnung (16) an der zweiten Seite (22) befindet.

4. Vorrichtung (10) nach einem der Ansprüche 2 bis 3, wobei der Vorratsbehälter (12) einen ersten Verbindungsbereich (31) aufweist und das Unterteil (15) einen korrespondierenden zweiten Verbindungsbereich (32) aufweist und der erste Verbindungsbereich (31) und der zweite Verbindungsbereich (32) mechanisch reversibel miteinander verbindbar sind.

5. Vorrichtung (10) nach einem der vorhergehenden Ansprüche, wobei die Öffnung (16) eine kreisrunde oder schlitzförmige Grundform aufweist.

6. Vorrichtung (10) nach einem der vorhergehenden Ansprüche, wobei am Abstreifer (18) Abstandhalter (40) angeordnet sind, um einen Abstand zwischen dem Abstreifer (18) und einem zu beschichtenden länglichen Implantat (5) zu gewährleisten.

7. Vorrichtung (10) nach dem vorhergehenden Anspruch, wobei die Abstandhalter (40) in Umfangsrichtung gleichmäßig verteilt angeordnet sind.

8. Vorrichtung (10) nach einem der Ansprüche 6 bis 7, wobei die Abstandhalter (40) stegförmig ausgestaltet sind und in einem Winkel (α) von weniger als 30° zu einer zentralen Achse (A) der Vorrichtung (10) verlaufen.

9. Vorrichtung (10) nach dem vorhergehenden Anspruch, wobei die stegförmigen Abstandhalter (40) einen Querschnitt mit einer Anlageseite (41) und einer freien Seite (42) aufweisen, wobei eine erste Breite (B1) der Abstandhalter (40) an der Anlageseite (41) zumindest bereichsweise größer ist als eine parallel zur ersten Breite (B1) gemessene zweite Breite (B2) der Abstandhalter (40) an der freien Seite (42).

10. Vorrichtung (10) nach einem der Ansprüche 8 bis 9, wobei die stegförmigen Abstandhalter (40) einen dreieckigen, trapezförmigen, rechteckigen oder halbkugelförmigen Querschnitt aufweisen.

11. Vorrichtung (10) nach einem der Ansprüche 8 bis 10, wobei ein zum Vorratsbehälter (12) hin weisendes Ende (44) der stegförmigen Abstandhalter (40) gerundet ausgeführt ist.

12. Vorrichtung (10) nach einem der vorhergehenden Ansprüche, wobei zumindest eine dem Unterteil (15) abgewandte Seite des Vorratsbehälters (12) als Zylinder (50) ausgebildet ist, wobei die Vorrichtung (10) ferner einen dem Zylinder (50) korrespondierenden Kolben (52) zum Pressen des Beschichtungsmittels (8) aufweist.

13. System, umfassend eine Vorrichtung (10) nach einem der Ansprüche 4 bis 12 sowie ein zusätzliches Unterteil (15) mit einer Öffnung (16), wobei das die Öffnung (16) ausbildende Material (17) gummielastisch ist und als Abstreifer (18) dient, wobei das zusätzliche Unterteil (15) einen zweiten Verbindungsbereich (32) aufweist, der mit dem ersten Verbindungsbereich (31) der Vorrichtung (10) korrespondiert und mechanisch reversibel mit diesem verbunden werden kann.

14. Verwendung einer Vorrichtung (10) gemäß einem der Ansprüche 1 bis 12 zur Beschichtung eines länglichen Implantats (5).

15. Verfahren zur Beschichtung eines länglichen Implantats (5) unter Verwendung einer Vorrichtung (10), umfassend Bereitstellen einer Vorrichtung (10) mit einem Vorratsbehälter (12), in dem ein Beschichtungsmittel (8) aufgenommen ist, Bewegen eines länglichen Implantats (5) durch das Beschichtungsmittel (8) sowie durch eine Öffnung (16) in einem Unterteil (15) der Vorrichtung (10), wobei überschüssiges Beschichtungsmittel (8) durch einen Abstreifer (18) der Vorrichtung (10) von dem Implantat (5) abgestreift wird.
